# EUROPÄISCHE PATENTSCHRIFT

(11) **EP 2 354 259 B1**
(45) Veröffentlichungstag und Bekanntmachung des Hinweises auf die Patenterteilung: **14.03.2018**
(21) Anmeldenummer: 11152764.4
(22) Anmeldetag: 31.01.2011
(51) Int. Cl.: C21C 7/10, F27D 3/16, C21C 5/46

(54) **Vakuumumlaufentgasungsanlage mit Zündbrenner**
Vacuum circulation gas removal assembly with ignitor
Installation de dégazage continu sous vide dotée d'un brûleur d'allumage

(30) Priorität: 05.02.2010 DE 102010007119
(43) Veröffentlichungstag der Anmeldung: 10.08.2011
(73) Patentinhaber: Tenova Metals Deutschland GmbH, 45141 Essen (DE)
(72) Erfinder: Schwarz, Jörg, 56477, Rennerod (DE)
(74) Vertreter: Viering, Jentschura & Partner mbB Patent- und Rechtsanwälte

(56) Entgegenhaltungen:
- WO-A1-00/68442
- DE-C1- 19 811 722
- JP-A- 8 325 630

## Beschreibung

Die Erfindung richtet sich auf eine Vakuumumlaufentgasungsanlage umfassend ein Vakuumgefäß mit einem an eine Vakuumpumpe anschließbaren Dom und zwei in die Schmelze einer zugeordneten Pfanne eintauchbaren Tauchrohren sowie mit einer in dem Vakuumgefäß verschiebbar positionierbaren Blaslanze mit kombinierter Sauerstoffaufblas- und Brennerfunktion, wobei der Blaslanze ein Zündbrenner zugeordnet ist.

Zum Behandeln von Metall-, insbesondere Stahlschmelzen durch Aufblasen von Sauerstoff auf die Oberfläche einer in einem Vakuumgefäß befindlichen Schmelze ist im Verlaufe der letzten Jahrzehnte die Vakuumbehandlung als sekundärmetallurgisches Verfahren immer weiterentwickelt worden. Die Vakuumbehandlung dient dazu, die Restgehalte an unerwünschten Begleitelementen in der Schmelze weitestgehend zu verringern, einen möglichst hohen Reinheitsgrad der Schmelze zu erreichen sowie die Zusammensetzung des Stahls möglichst genau einzustellen und die Homogenität des erstarrten Stahls zu verbessern. Für das Vakuumfrischen steht als Vakuumbehandlung unter anderem die sogenannte Umlauf-Entgasung, häufig auch als RH-Verfahren bezeichnet, zur Verfügung. Bei diesem Verfahren wird unter anderem gasförmiger Sauerstoff mit Hilfe einer wassergekühlten Lanze auf die im Vakuumgefäß befindliche Schmelze geblasen, wobei unter Vakuum dann metallurgische Prozesse ablaufen, die zu einer Qualitätsverbesserung des behandelten Flüssigstahls führen. Kernstück einer solchen Anlage ist ein Vakuumgefäß mit einer an eine Vakuumpumpe angeschlossenem Dom. Mit zwei Tauchrohren taucht das Vakuumgefäß in die flüssige Schmelze einer Pfanne ein, wobei nach Erzeugung eines Vakuums ein Umlauf der Schmelze von der Pfanne durch ein Tauchrohr in das Vakuumgefäß und durch das andere Tauchrohr zurück in die Schmelze erzeugt wird. Das Vakuumgefäß muss vor Durchführung einer Behandlung der Schmelze jeweils aufgewärmt werden, zwischen Behandlungszyklen in den Behandlungspausen warmgehalten und ggf. nach Behandlungsende gereinigt werden. Um mit einer Lanze sowohl das Sauerstoffaufblasen auf die Schmelze im Vakuumgefäß durchführen zu können, als auch mit dieser Lanze die notwendige Beheizung und Erwärmung des Vakuumgefäßes durchführen zu können, werden heutzutage üblicherweise Blaslanzen mit kombinierter Sauerstoffaufblas- und Brennerfunktion verwendet, durch welche hindurch sowohl ein Brenngas ausgestoßen und im Mündungsbereich der Blaslanze zu einer Flamme gezündet werden kann als auch ein Sauerstoffstrahl auf die Schmelze geblasen werden kann. Die Blaslanze ist in dem Vakuumgefäß axial verschiebbar angeordnet und dabei durch eine vakuumdichte Durchführung im Dom geführt. Üblicherweise ist eine solche Kombi-Blaslanze mit einem integrierten Zündbrenner ausgestattet. Derartige Kombi-Lanzen dürfen ab einer bestimmten Leistung nicht direkt gezündet werden, sondern müssen durch einen Zündbrenner gezündet werden, der nach Zünden seiner Zündflamme und nach Erkennen dieser Zündflamme Brennstoffgas und Sauerstoff in der Kombi-Blaslanze zur Zündung der Hauptflamme freigibt. Bei den bekannten Kombi-Blaslanzen ist dieser Zündbrenner unmittelbar an der Spitze der Kombi-Blaslanze in unmittelbarer Nähe der Gas- und Sauerstoffaustritte für die Hauptflamme ausgebildet. Der Zündbrenner ist daher den Umgebungsbedingungen, in die sich die Kombi-Blaslanze begibt, unmittelbar ausgesetzt. In der Sauerstoffaufblasfunktion der Kombi-Lanze führt dies dazu, dass Stahl- und/oder Schlackespritzer in den oder an den Zündbrenner gelangen können. Dies führt häufig zu einem Ausfall des Zündbrenners und damit zu einem Ausfall der Brennerfunktion der Kombi-Lanze, die dann zeitaufwändig gereinigt und/oder gewechselt werden muss, was bei entsprechendem Produktionsausfall äußerst kostspielig ist.

Eine gattungsgemäße Vakuumumlaufentgasungsanlage ist aus der DE 600 08 959 T2 sowie dem Prospekt "Secondary metallurgy solutions" der Siemens VAI Metals Techologies GmbH bekannt.

Diese daraus bekannten Vakuumumlaufentgasungsanlagen weisen die vorstehend dargelegten Nachteile auf.

Aus der DE 198 11 722 C1 ist eine Vakuumumlaufentgasungsanlage mit einer kurzen Sauerstofflanze bekannt, die außerhalb des Vakuumgefäßes einen Gasbrenner aufweist, der sich durch einfaches Verschwenken um eine Achse mit der Lanze kuppeln lässt. Ebenso offenbart dieses Dokument eine Vakuumumlaufentgasungsanlage, bei welcher mit Abstand zu der kurzen Sauerstofflanze in der Seitenwand des Vakuumgefäßes der Vakuumumlaufentgasungsanlage ein Gasbrenner angeordnet ist. Die Sauerstofflanze kann auch als Kombilanze zusätzlich mit einer Brennerfunktion ausgestattet sein.

Blaslanzen mit kombinierter Sauerstoffaufblas- und Brennerfunktion sind zudem aus den in der JP 8 325 630 A und der WO 00/68442 A1 offenbarten Vakuumumlaufentgasungsanlagen bekannt.

Der Erfindung liegt die Aufgabe zugrunde, eine Lösung zu schaffen, die es ermöglicht, eine erhöhte Blaslanzen- und Anlagenverfügbarkeit bei einer Vakuumumlaufentgasungsanlage zu erzielen.

Bei einer Vakuumumlaufentgasungsanlage der eingangs näher bezeichneten Art wird diese Aufgabe erfindungsgemäß dadurch gelöst, dass der Zündbrenner in einer Zündkammer angeordnet ist, in welcher er insbesondere durch Verfahren der Blaslanze in verschiedene Relativlagen zur Blaslanze bringbar ist.

Der Zündbrenner wird erfindungsgemäß in einer vorzugsweise außerhalb des eigentlichen metallurgischen Behandlungsraumes des Vakuumgefäßes der Vakuumumlaufentgasungsanlage ausgebildeten Zündkammer angeordnet, durch welche die Kombi-Blaslanze hindurchbewegbar ist. Auf diese Weise lässt sich der Zündbrenner insbesondere durch Verfahren der Blaslanze in verschiedene Relativlagen zur Blaslanze bringen. Dies ermöglicht es, den Zündbrenner in einem von den übrigen Umgebungsbedingungen innerhalb des Vakuumgefäßes geschützten und in Relation zu den innerhalb des Vakuumgefäßes entstehenden Temperaturen mäßig warmen Raum anzuordnen, in welchem der Zündbrenner nur bis zur Zündung der Hauptflamme der Blaslanze einer erhöhten Temperatur ausgesetzt ist. Hierzu wird die Blaslanze zunächst in eine Zündposition innerhalb der Zündkammer hochgefahren, in welcher sie oberhalb des Zündbrenners positioniert ist. Nach Erkennung der Zündflamme des Zündbrenners wird dann durch eine übliche Flammenüberwachung die notwendige minimale Brenngaszufuhr und Sauerstoffzufuhr in die Blaslanze zur Zündung der Hauptflamme der Blaslanze mit Minimallast freigegeben. Sobald ein Sensor das Zünden und Brennen der Hauptflamme der Blaslanze erkennt, wird die Zündflamme des Zündbrenners ausgeschaltet und die Blaslanze in ihren Arbeitsbereich innerhalb des Vakuumgefäßes abgesenkt. In dieser abgesenkten Arbeitsposition wird dann die Brennstoffgas- und Sauerstoffzufuhr soweit gesteigert und derart eingestellt bis die Blaslanze dann in ihrer Brennerfunktion mit Nennlast oder Maximallast betrieben wird.

Dadurch, dass der Zündbrenner und die Blaslanze getrennt voneinander angeordnet und ausgebildet sowie in unterschiedliche Relativpositionen zueinander bringbar sind, wird es vermieden, dass es zu einem Ausfall der Blaslanze und damit der Anlage aufgrund den Zündbrenner erreichender Stahl- oder Schlackenspritzer kommt. Die Blaslanzen-, und damit Anlagenverfügbarkeit, wird dadurch erhöht. Außerdem ergeben sich kürzere Lanzen- und Zündbrennerwechselzeiten, da zum Lanzenwechseln kein in der Blaslanze integrierter Zündbrenner mit aus dem Vakuumgefäß herausgefahren werden muss, wozu dieser von Gas- und Sauerstoffzuführleitungen ggf. abgetrennt werden müsste. Andererseits lässt sich der Zündbrenner bei Ausfall wechseln, ohne dass dafür die Blaslanze bewegt oder aus dem Vakuumgefäß herausgefahren werden muss. Schließlich ergeben sich für die Blaslanze geringere Herstellkosten, da in diese kein Zündbrenner und diesem zuzuordnende Kühlvorrichtungen vorgesehen werden müssen. Da die Blaslanze nicht mehr mit einem integrierten Zündbrenner versehen ist, lässt sich dadurch der Lanzendurchmesser verringern, was einen verringerten Kühlwasserbedarf und damit einen geringeren Verbrauch an Betriebsmittel mit sich bringt. Außerdem ist nur noch eine geringere Antriebsleistung für das Auf- und Abfahren der Blaslanze erforderlich, da diese dann auch ein geringeres Gewicht aufweist.

Eine besonders zweckmäßige Anordnung der Zündkammer ist die Platzierung oben auf dem Dom des Vakuumgefäßes auf der dem Vakuumgefäßinnenraum abgewandten Seite. Hierdurch ist es möglich, die senkrecht oder vertikal in dem Vakuumgefäß verfahrbare Blaslanze ohne wesentliche Veränderungen am üblichen Mechanismus zur Verfahrbarkeit der Lanze vornehmen zu müssen in den Bereich des Zündbrenners zu verfahren. Es ist aber auch möglich, die Zündkammer am Dom oder am RH-Gefäß bzw. am Vakuumgefäß anzuordnen. Die Erfindung sieht daher weiterhin vor, dass die Zündkammer auf dem Dom oder am Vakuumgefäß ausgebildet ist.

Ganz besonders zweckmäßig lässt sich hierbei auch die vakuumdichte Durchführung der Blaslanze in das Vakuumgefäß hinein im Bereich der Zündkammer vorsehen. Die Erfindung zeichnet sich schließlich auch dadurch aus, dass an der Zündkammer eine vakuumdichte Durchführung der Blaslanze ausgebildet ist.

Die Erfindung ist nachstehend anhand der Zeichnung beispielhaft näher erläutert. Diese zeigt in
- Fig. 1: in schematischer Schnittdarstellung eine Vakuumumlaufentgasungsanlage mit einer Zündkammer und in
- Fig. 2: in schematischer Teilschnittdarstellung eine Zündkammer mit Zündbrenner.

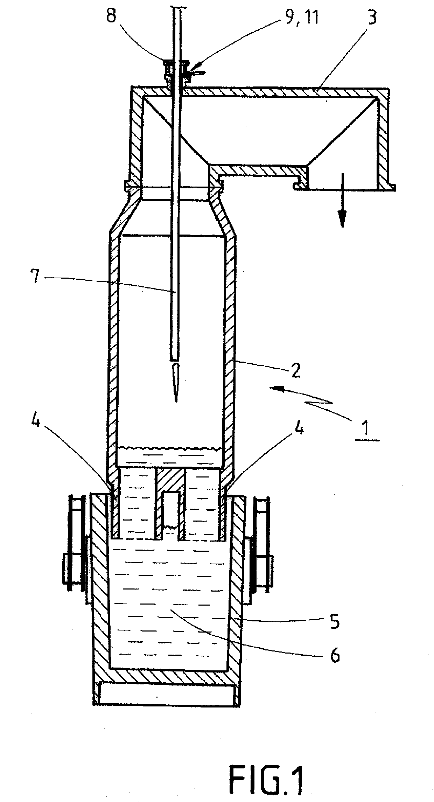

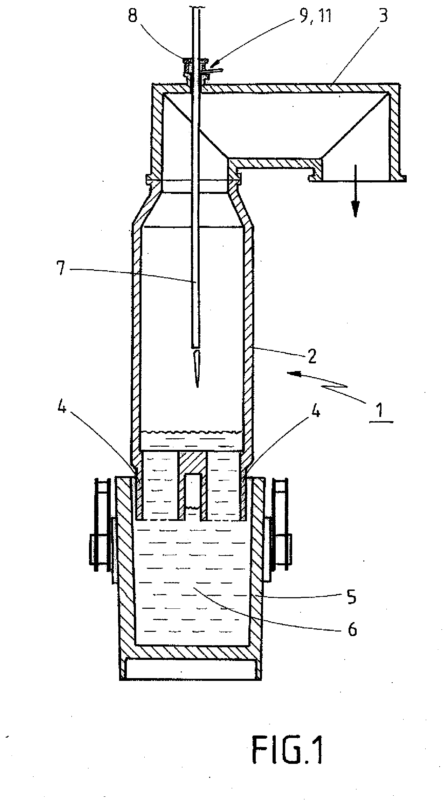

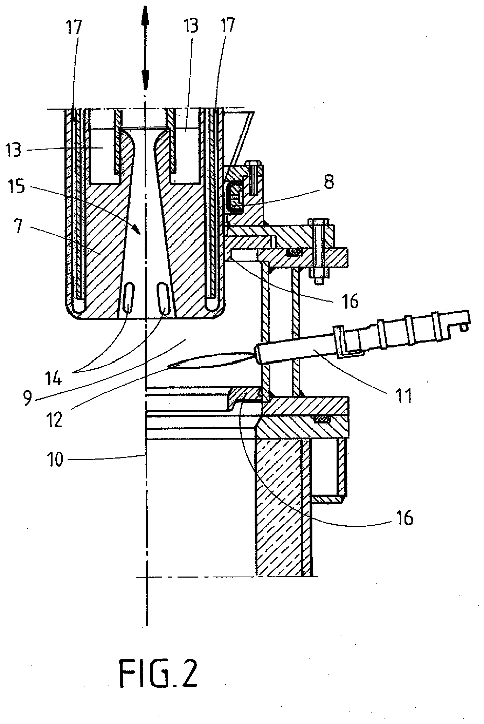

Die Fig. 1 zeigt eine insgesamt mit 1 bezeichnete Vakuumumlaufentgasungsanlage. Diese besteht aus einem Vakuumgefäß 2 oder RH-Gefäß, das in seinem oberen Teil in einem zu einer Vakuumpumpe führenden Dom 3 übergeht. Ferner ist das Vakuumgehäuse 2 mit zwei Tauchrohren 4 ausgestattet, die in eine in einer Pfanne 5 befindliche Schmelze 6 eintauchen. Durch eines der Tauchrohre 4 steigt die Schmelze 6 aus der offenen Pfanne 5 in das Vakuumgehäuse 2 hinein und durch das andere Tauchrohr 4 fließt die Schmelze 6 wieder in die Pfanne 5 zurück. Im Vakuumgehäuse 2 wird die Schmelze 6 bei angelegtem Vakuum einer metallurgischen Behandlung unterworfen. Insbesondere wird die Schmelze 6 beim Vakuumfrischen einem durch eine Blaslanze 7 ausgeblasenen Sauerstoffstrom ausgesetzt. Bei der Blaslanze 7 handelt es sich um eine Blaslanze die als Kombi-Blaslanze sowohl mit einer Sauerstoffaufblasfunktion zur Abgabe von Sauerstoff als auch mit einer Brennerfunktion ausgestattet ist. Die Blaslanze 7 ist im Vakuumgehäuse 2 vertikal verschiebbar angeordnet und in einer vakuumdichten Durchführung 8, die meist als Blähdichtung ausgeführt ist, geführt. Außerhalb der Haube oder des Doms 3 ist auf dieser oberseitig eine Zündkammer 9 ausgebildet, in welcher die Blaslanze 7 zentral geführt ist. In der Fig. 2 ist in Schnittdarstellung die Spitze der Blaslanze 7 dargestellt, wobei die übrigen im Wesentlichen zylinderförmig ausgebildeten Bauteile der Zündkammer und des Domstutzens nur auf einer Seite der Längsachse 10 dargestellt sind. Mit der Längsachse 10 als Spiegelachse wiederholen sie sich aber auch auf der anderen Seite. In der in Fig. 2 dargestellten Position befindet sich die Blaslanze 7 mit ihrer Mündungsöffnung innerhalb der Zündkammer 9. In die Zündkammer 9 mündet ein Zündbrenner 11 mit dessen Zündflamme 12 das Brenngassauerstoffgemisch der Hauptflamme, welches aus der Blaslanze 7 austritt, gezündet wird. Hierbei wird das Brennstoffgas über Kanäle 13 mit Austrittsöffnungen 14 und wird der Sauerstoff zentral über eine Mündungsöffnung 15 zugeführt und abgegeben. Nach Zündung der Hauptflamme wird die Blaslanze 7 vertikal durch die Zündkammer 9 hindurch nach unten in den Innenraum des Vakuumgefäßes 2 geführt, so dass dort die Hauptflamme dann auf Volllast oder Nennlast hochgefahren werden und ihre wärmeabgebende Funktion übernehmen kann. In ihrer Sauerstoffaufblasfunktion wird aus der Mündungsöffnung 15 der Blaslanze 7 kein Brennstoffgas sondern ausschließlich Sauerstoff zur metallurgischen Behandlung der Schmelze 6 ausgestoßen und auf die Oberfläche der Metallschmelze 6 innerhalb des Vakuumgefäßes 2 aufgeblasen. Im Bereich ihrer Außenumfangswand weist die Blaslanze 7 übliche Kühlwasserkanäle 17 auf. Aufgrund der vertikalen Bewegbarkeit und Verschiebbarkeit oder Verfahrbarkeit der Blaslanze 7 im Vakuumgefäß 2 und in der Zündkammer 9 ist der Zündbrenner 11 in die verschiedenen Relativlagen zur Blaslanze 7 bringbar. Neben der die vakuumdichte Durchführung 8 ausbildenden, ringförmigen Dichtung sind in der Zündkammer 9 auch noch zwei an der Außenwand der Blaslanze 7 anliegende Abstreifer 16 ausgebildet.

## Patentansprüche

1. Vakuumumlaufentgasungsanlage (1) umfassend ein Vakuumgefäß (2) mit einem an eine Vakuumpumpe anschließbaren Dom (3) und zwei in die Schmelze (6) einer zugeordneten Pfanne (5) eintauchbaren Tauchrohren (4) sowie mit einer in dem Vakuumgefäß (2) verschiebbar positionierbaren Blaslanze (7) mit kombinierter Sauerstoffaufblas- und Brennerfunktion, wobei der Blaslanze (7) ein Zündbrenner (11) zugeordnet ist, **dadurch gekennzeichnet,**
**dass** der Zündbrenner (11) in einer Zündkammer (9) angeordnet ist, in welcher er insbesondere durch Verfahren der Blaslanze (7) in verschiedene Relativlagen zur Blaslanze (7) bringbar ist.

2. Vakuumumlaufentgasungsanlage (1) nach Anspruch 1, **dadurch gekennzeichnet, dass** die Zündkammer (9) auf dem Dom (3) oder am Vakuumgefäß (2) ausgebildet ist.

3. Vakuumumlaufentgasungsanlage (1) nach Anspruch 1 oder 2, **dadurch gekennzeichnet, dass** an der Zündkammer (9) eine vakuumdichte Durchführung (8) der Blaslanze (7) ausgebildet ist.

## Claims

1. Vacuum circulation degassing system (1), comprising a vacuum vessel (2) with dome (3) connectable to a vacuum pump and two immersible tubes (4) that can be submersed in the melt (6) of an associated ladle (5), and with a blower lance (7) displaceably positionable in the vacuum vessel (2) with a combined oxygen blowing and burner function, wherein an ignition burner (11) is allocated to the blower lance (7),
**characterised in that**
the ignition burner (11) is arranged in an ignition chamber (9), in which it can be brought into various positions relative to the blower lance (7), in particular through displacing the blower lance (7).

2. Vacuum circulation degassing system (1) according to claim 1, **characterised in that** the ignition chamber (9) is formed on the dome (3) or on the vacuum vessel (2).

3. Vacuum circulation degassing system (1) according to claim 1 or 2, **characterised in that** a vacuum-tight passage (8) of the blower lance (7) is formed on the ignition chamber (9).

## Revendications

1. Installation de dégazage par circulation de vide (1) comprenant un récipient sous vide (2) avec un dôme (3) pouvant être connecté à une pompe à vide et deux tubes plongeurs (4) pouvant être plongés dans la matière en fusion (6) d'une poche de coulée (5) correspondante, ainsi qu'avec une lance de soufflage (7) pouvant être positionnée de manière mobile dans le récipient sous vide (2), avec une fonction combinée de soufflage d'oxygène et de brûleur, un brûleur d'allumage (11) correspondant à la lance de soufflage (7),
**caractérisée en ce que**
le brûleur d'allumage (11) est disposé dans une chambre d'allumage (9) dans laquelle il peut être mis dans différentes positions relatives par rapport à la lance de soufflage (7) par déplacement de la lance de soufflage (7).

2. Installation de dégazage par circulation de vide (1) selon la revendication 1, **caractérisée en ce que** la chambre d'allumage (9) est réalisée sur le dôme (3) ou sur le récipient sous vide (2).

3. Installation de dégazage par circulation de vide (1) selon la revendication 1 ou 2, **caractérisée en ce que**, sur la chambre d'allumage (9) est réalisé un passage étanche au vide (8) de la lance de soufflage (7).
